# FASCICULE DE BREVET EUROPEEN

(11) **EP 0 941 364 B1**
(45) Date de publication et mention de la délivrance du brevet: **05.04.2006**
(21) Numéro de dépôt: 97913233.9
(22) Date de dépôt: 31.10.1997
(51) Int. Cl.: C12Q 1/68, C12N 15/11, C12N 15/12, A61K 48/00

(54) **PROCEDE DESTINE A METTRE EN EVIDENCE L'ETAT D'UNE CELLULE MALIGNE ET PROCEDE DE TRAITEMENT**
VERFAHREN ZUR BESTIMMUNG DES MALIGNEN ZELLEZUSTANDS UND VERFAHREN ZUR BEHANDLUNG
PROCESS AIMED AT EVIDENCING THE STATE OF A MALIGNANT CELL AND PROCESS FOR TREATMENT

(30) Priorité: 31.10.1996 FR 9613326
(43) Date de publication de la demande: 15.09.1999
(73) Titulaire: Cerenis, 75011 Paris (FR)
(72) Inventeur: Calvo, Fabien, 75004 Paris (FR)
(74) Mandataire: Warcoin, Jacques
(86) Numéro de dépôt international: PCT/FR1997/001960
(87) Numéro de publication internationale: WO 1998/018960

(56) Documents cités:
- EP-A- 0 500 290
- WO-A-94/19492
- WO-A-95/09928
- WO-A-95/13375
- WO-A-96/25507
- US-A- 5 264 343
- WALDMAN T ET AL: "Uncoupling of S phase and mitosis induced by antcancer agents lacking p21" NATURE, vol. 381, juin 1996, pages 713-16, XP002035345 cité dans la demande
- HAAG M ET AL: "Reverse transformation and genome exposure in the C6 glial tumor cell line" CANCER INVESTIGATION, vol. 12, no. 1, 1994, pages 33-45, XP002035346
- NEMANI M ET AL: "Activation of the human homologue of the Drosophila sina gene in apoptosis and tumor suppression" PROC. NATL. ACAD. SCI. USA, vol. 93, août 1996, pages 9039-42, XP002035347 cité dans la demande

## Description

La présente invention concerne, de façon générale, la mise en évidence d'une réorganisation du noyau durant la suppression tumorale et les conséquences qui en découlent, tant au niveau du diagnostic que de la thérapie, notamment anticancéreuse.

De plus en plus d'indices suggèrent que la régulation de l'expression des gènes, qu'il s'agisse de leur activation ou de leur mise en silence, se déroule en deux étapes dans lesquelles on observe, tout d'abord, une compartimentalisation des chromosomes dans le noyau avant la transcription.

La présente invention a mis en évidence le fait que, dans des tissus cancéreux, cette compartimentalisation se faisait différemment par rapport à une cellule normale, ce qui pouvait constituer un élément de diagnostic.

Cette observation a pu être réalisée notamment en étudiant le mécanisme du gène p21WAF1 et de son comportement lors de l'activation qui contribue à la suppression du processus tumorigène.

Ce phénomène a pu être mis en évidence notamment grâce à un perfectionnement du procédé à la DNase I qui a permis de suivre ce processus.

Le procédé à la DNase I repose sur l'observation que la chromatine active au niveau de la transcription est plus particulièrement sensible à l'action des nucléases. Elle contient en particulier des sites hypersensibles à la DNase I (il s'agit de segments d'ADN relativement courts qui sont très sensibles à un grand nombre d'agents de clivage). Ces segments d'ADN exempts de nucléosomes sont associés avec différentes fonctions de l'expression du gène. De telles "régions de chromatine ouvertes" peuvent permettre la fixation de facteurs spécifiques et possèdent d'importantes fonctions de régulation, on dit alors que l'ADN marqué est l'ADN exposé.

On a donc pu étudié, grâce à ce procédé, les relations possibles entre la transcription des gènes et leur hypersensitivité à la DNase I in situ, notamment comme marqueur permettant l'analyse de l'organisation du génome global dans les phénomènes cancéreux.

Le procédé général qui sera appelé ci-après "procédé à la DNase" est décrit notamment dans le brevet Américain publié US-5-264-343, ce document est cité ici à titre de référence explicite et les informations qu'il contient ne seront pas reprises plus en détail. On pourra également se référer à la publication de Puck et al (Puck T.T., Bartholdi M, Krystosek A., Johnson R. & M Haag. Somatic Cell and Molec. Genetics 17,489-503, 1991).

Le document WO 95/09928 est relatif à une méthode de détection de cancer par mise en évidence de délétion chromosomique.

Toutefois, le procédé décrit dans le brevet en question ne permet pas l'analyse de tissus fixés par la méthode formol et paraffine, c'est-à-dire des tissus obtenus par la méthode mise en oeuvre dans la plupart des laboratoires d'anatomie pathologie. En effet, le procédé décrit au brevet Américain concerne essentiellement l'analyse de systèmes en culture cellulaire.

C'est donc l'un des objets de la présente invention de proposer un nouveau procédé à la DNase permettant d'effectuer des diagnostics sur des tissus fixés.

Mais, de plus, la présente invention a pour objet, de façon générale, des procédés destinés à mettre en évidence l'état d'une cellule maligne dans lesquels on met en évidence la conformation précise d'au moins un gène au sein du noyau de ladite cellule par marquage dudit gène in situ et évaluation de l'écart de cette conformation par rapport à une cellule normale comme index de sa malignité.

En effet, si la mise en évidence de la position de l'ADN exposé par la méthode à la DNase permet une analyse globale de l'état de malignité d'un tissu, par contre, l'étude de la conformation précise d'au moins un gène au sein d'un chromosome et la mesure de son écart à la normalité permet, non seulement un classement des différents types de cellules malignes, mais également une évaluation de leur index de malignité et bien entendu, par là même, le suivi d'un traitement thérapeutique.

Il convient de rappeler que, dans ce qui va suivre, on utilisera éventuellement la terminologie de "maligne" ou "d'index de malignité" également pour rendre compte de phénomènes non directement liés à l'oncogénèse mais caractérisés également par une désorganisation des chromosomes au sein du noyau, par exemple la fibrose cystique.

Ce second procédé repose, là encore, sur la mise en évidence du fait qu'en évaluant la conformation, c'est-à-dire à la fois la position mais également, si nécessaire, la structure d'un ou plusieurs gènes par toute technique appropriée, notamment par hybridation in situ par fluorescence par exemple (FISH), on constate que, suivant l'état de malignité de la cellule, certains gènes impliqués dans la suppression et/ou l'activation tumorale se déplacent du centre du noyau vers l'extérieur et que leur écart à la normalité constitue un bon indice de l'état de la cellule qui est appelé ci-après "index de malignité", cet index pouvant être évalué par toute méthode appropriée entre une cellule normale et une cellule dont la malignité sera considérée comme irréversible.

Ce procédé permet une classification des tumeurs en fonction de la position des différents gènes dans le noyau et même de la structure tridimensionnelle de ces gènes spécifiques dans le noyau.

Dans un premier temps, il sera possible d'étudier la position des gènes dans le noyau simplement en étudiant la position des centromères de chromosome qui les portent.

Ce procédé peut être mis en oeuvre avec des sondes d'hybridation de type sonde centromérique, dans ce cas la normalité correspond à une localisation périphérique des chromosomes portant les sondes centromériques et en particulier, comme cela sera développé ci-après, ces sondes centromériques pourront correspondre aux chromosomes 13q et 16q, lesquels sont porteurs de gènes HUMSIAH et Rbr-2 colocalisés sur le chromosome 16q et le gène Rb localisé sur le chromosome 13q, ces gènes étant impliqués dans le phénomène de suppression cancéreuse. Il est bien entendu que les sondes centromériques, utilisables dans les procédés selon l'invention, peuvent correspondre à tout autre chromosome cellulaire, de la même manière que le marquage de gène, selon les procédés de l'invention, peut correspondre à un ou plusieurs gènes portés sur un ou plusieurs chromosomes choisis parmi l'ensemble des chromosomes de la cellule.

Mais, comme cela a été indiqué, la présente invention concerne également un procédé destiné à mettre en évidence l'état global de malignité des cellules d'un tissu après fixation, caractérisé en ce qu'on marque l'ADN nucléaire exposé et on marque l'ensemble de l'ADN avec un marqueur spécifique de l'ADN, ces deux marqueurs étant capables d'émettre des signaux spécifiques non contaminés.

Par "signaux spécifiques non contaminés" on entend qu'il est possible de visualiser séparément sans contamination notable les deux signaux correspondant aux deux marqueurs.

De façon générale, l'ADN nucléaire exposé est marqué par la méthode à la DNase I, comme cela a été décrit précédemment, et l'ADN est marqué par le marqueur spécifique chromomycine A-3 (CA-3).

Parmi les marqueurs de l'ADN nucléaire exposé il faut citer plus particulièrement la tétraméthyl rhodamine utilisée en combinaison avec la peroxydase. Le marquage à la peroxydase peut s'effectuer par toute méthode appropriée, notamment des couplages de type avidine ou streptovidine/biotine ou DIG/antiDIG (DIG pour digoxigénine).

En effet, le substrat de la peroxydase, dénommé tyramide- TRITC, permet d'amplifier le signal obtenu d'un facteur 15 et permet ainsi d'analyser une distribution fine du signal à l'intérieur du noyau.

L'intérêt d'utiliser un marqueur spécifique de l'ADN est qu'il permet d'analyser de façon semi-automatique le cycle cellulaire, donc de faire une corrélation entre l'exposition du génome obtenu grâce à la méthode à la DNase I et les phases du cycle cellulaire.

Ainsi, la présente invention concerne également un procédé selon l'invention , caractérisé en ce qu'on évalue la position de l'ADN nucléaire exposé par rapport à la phase du cycle cellulaire mise en évidence par le marqueur spécifique de l'ADN comme index de l'état de malignité des cellules de tissus.

En outre, les deux fluorochromes ainsi utilisés ont une émission dans la même zone du spectre lumineux, c'est-à-dire 600 nm mais leur excitation spécifique est très différente, 467 nm pour la CA-3 et 514 nm pour la TRITC, de même que les coefficients d'extinction, ce qui permet d'obtenir des signaux spécifiques sans aucune contamination entre les deux marqueurs. En outre, grâce à ces marqueurs, l'analyse peut se faire avec un seul laser à argon qui est le laser le plus répandu en microscopie

Ce procédé selon la présente invention permet d'utiliser des tissus fixés dans le formol selon des méthodes de routine et permet ainsi une analyse de tissus provenant, par exemple, d'une collection de tissus.

De façon générale, les méthodes ainsi décrites permettent de diagnostiquer les états malins à partir de tissus et/ou de cultures cellulaires et permettent, grâce à la classification établie, un facteur de pronostic en pathologie diagnostique.

En particulier, ils permettent de faire la différence, par exemple, entre des hyperplasies simples (sein, prostate) qui sont des pathologies bénignes, des hyperplasies atypiques (sein, prostate) à évolution variable difficile à établir et qui constituent des pathologies connues comme étant "border line", mais que dans le cas présent on pourra plus facilement classifier grâce aux différents éléments de marquage qui ont été évoqués, et le carcinome in situ

Ces procédés permettent également la mise en place rapide de modèles permettant de tester des produits anticancéreux, et notamment, en étudiant leur action sur la réorganisation de certains chromosomes spécifiques, une classification plus fine de l'activité des différents agents en cause

De façon préférée, on met en oeuvre, afin de tester les produits anticancéreux ou apparentés, les procédés décrits précédemment dans lesquels on met en évidence l'état d'une cellule maligne, puis on traite ladite cellule avec un produit anticancéreux ou apparenté et on évalue l'activité dudit composé en mesurant la variation de l'index de malignité.

L'invention comprend les produits anticancéreux ou apparentés, caractérisés en ce qu'ils ont été sélectionnés par un procédé selon l'invention.

Par produits apparentés, on entend désigner les produits agissant dans des pathologies non directement liées à l'oncogenèse et telles que définies précédemment

Bien entendu, par là même, ces procédés permettront un suivi thérapeutique en cas de traitement.

Enfin, la présente invention concerne un procédé de traitement de cellules présentant une désorganisation au niveau du noyau et l'application de ce procédé dans le traitement de certaines pathologies, notamment tumorales.

La présente invention repose, en effet, sur la mise en évidence des propriétés de réorganisation du noyau associé au gène p21WAF1.

L'association de p21WAF1 avec le signal de p53, son rôle comme inhibiteur des kinases dépendant des cyclines (CDKs) et dans le contrôle de l'endoréplication chromosomique indiquent que ce gène joue un rôle majeur dans le contrôle de la croissance cellulaire

Il a maintenant été mis en évidence que la réexpression de p21WAF1 dans un modèle conduisait à une suppression de la tumeur.

C'est pourquoi la présente invention concerne un procédé de traitement de cellules présentant une désorganisation du noyau, caractérisé en ce qu'on provoque l'expression du produit du gène p21WAF1 ou d'un produit équivalent ou bien on introduit le produit du gène ou le produit équivalent

Le gène p21WAF1 est connu et a déjà été décrit à plusieurs reprises, on pourra se reporter, pour sa caractérisation, son isolement ou certaines de ses propriétés, aux documents ci-après et à leurs références :
■ Weinberg R.A Science 254, 1138-1146 (1992)
■ Telerman A. et al. Proc. Natl. Acad. Sci. USA 90, 8702-8706 (1993)
■ Amson R.B. et al. Proc. Natl. Acad. Sci. USA 93,3953-3957 (1996)
■ Nemani M. et al. Proc. Natl. Acad. Sci. USA 93, 9039-9042 (1996)
■ El-Deiry W.S. et al. Cell 75, 817-825 (1993)
■ Harper J.W., Adami G.R., Wei N., Keyomarsi K. & Elledge S J. Cell 75, 805-816 (1993)
■ Waga S., Hannon G.J., Beach D. & Stillman B. Nature 369, 574-578 (1994)
■ Yang Z.Y., Perkins N.D., Ohno T., Nabel E.G. & Nabel G.J Nature Medicine 1, 1052-1056 (1995).
■ Xiong Y. et al., Nature 366, 701-704 (1993)
■ Waldman T., Lengauer C., K W., K. & Vogelstein B. Nature 381, 713-716 (1996).

Comme cela sera explicité ci-après, la désorganisation du noyau peut être d'origine tumorale, mais il peut s'agir de désorganisations d'un autre type Les procédés de traitement de cellules selon l'invention, caractérisés en ce que la désorganisation du noyau est due à un phénomène tumoral ou non tumoral, font également partie de l'invention..

Par "désorganisation du noyau" on entend un noyau dans lequel le matériel génétique, en particulier les chromosomes, occupe une place différente de celle qu'il occupe dans le noyau de la cellule à l'état normal, désorganisation qui peut, notamment, être mise en évidence par les procédés décrits précédemment.

Bien que le phénomène de désorganisation du noyau ait été plus particulièrement mis en évidence dans les phénomènes tumoraux, cette désorganisation peut également intervenir dans d'autres phénomènes de types non tumoraux, comme la fibrose cystique (CFTR).

Le procédé selon la présente invention permet ainsi, soit par réinduction de l'expression du gène correspondant p21WAF1, soit par utilisation d'un produit du gène ou d'un produit équivalent, c'est-à-dire ayant la même activité de réorganisation que le produit du gène p21WAF1, de réorganiser le matériel génétique du noyau.

Bien entendu, l'induction de l'expression du gène peut être remplacée par un blocage de la répression du même gène ou d'un produit d'expression.

L'expression du gène p21WAF1 ou d'un produit équivalent peut être effectuée par tout procédé connu, dans les exemples, en particulier, on utilisera un vecteur d'expression d'origine virale dans lequel a été introduit le gène correspondant sous le contrôle d'un promoteur s'exprimant dans les cellules, cette introduction, que le vecteur reste non chromosomique ou qu'il s'intègre dans les chromomes, conduit à l'expression du produit de p21WAF1 et à la réduction de la malignité des cellules. Parmi les vecteurs viraux il faut citer, notamment, les vecteurs à adénovirus, à rétrovirus, à virus herpès ou même des systèmes de type synthétique ou même des ADN nus.

Bien que l'on puisse exprimer le gène p21WAF1 dans son entier, certaines versions tronquées, délétées ou mutées peuvent présenter des propriétés équivalentes et donc font également partie du procédé selon la présente invention.

Bien entendu, on peut utiliser les produits du gène p21WAF1 obtenus, par exemple, par culture cellulaire, de même que des cultures produisant, comme cela a été dit précédemment, des produits équivalents.

Il est également possible d'envisager une réactivation in situ du promoteur de p21WAF1 naturel, ou bien le blocage des systèmes de répression de son expression in situ.

De façon générale, la présente invention concerne l'utilisation du produit d'expression du gène p21WAF1 ou d'un produit équivalent ou bien d'un composé assurant l'expression dudit produit pour la réalisation d'un médicament destiné à assurer la réorganisation du noyau cellulaire des cellules dans des processus cancéreux ou de type non cancéreux avec désorganisation du noyau, ainsi qu'une composition thérapeutique caractérisée en ce qu'elle comporte, à titre de principe actif, le produit d'expression du gène p21WAF1 ou un produit équivalent ou bien un composé assurant l'expression dudit produit.

D'autres caractéristiques et avantages de la présente invention apparaîtront à la lectures des exemples ci-après qui seront décrits en se référant aux figures annexées et dont les légendes sont ci- dessous décrites:

### Figure 1 :

La figure 1 représente l'expression différentielle d'ARNm dans les cellules U937 transfectées témoin (pRSV) et exprimant p21WAF1 (pRSV-p21). L'analyse par Northern blot indique l'activation de p21WAF1, HUMSIAH, Rb, et Rbr-2 dans les cellules pRSV-p21 transfectées comparée aux cellules témoin pRSV transfectées avec le vecteur seul. GAPDH est utilisé comme sonde de contrôle.
Figure 2 : Tumorigénicité des cellules U937 pRSV témoin transfectées avec le vecteur seul comparée aux cellules pRSV-p21 transfectées
   La tumorigénicité est évaluée après injection sous-cutanée de 10⁷ cellules dans les souris scid/scid.
Figure 3: Repositionnement du chromosome 16 dans les cellules transfectées p21WAF1 U937.
   et b) : Hybridation par fluorescence in situ (FISH) utilisant une sonde centromérique alphoïde de chromosome 16, les 2 ou 3 points (normalement en rouge), apparaissant à l'intérieur du noyau (normalement en bleu), représentent les signaux de fluorescence correspondant à la sonde centromérique marquée. a) correspond aux noyaux de cellules U937 transfectées témoin (pRSV) et b) correspond aux noyaux de cellules transfectées p21WAF1 U937 (p21-WAF1). Le noyau des cellules transfectées U937 témoin et les cellules transfectées p21-WAF1 sont marqués au DAPI (bleu). Le signal FISH est révélé par la tyramide-TRITC (rouge) Trois spots sont ici visibles comte tenu d'une trisomie 16 de l'échantillon.
   c) Agrandissement d'une cellule transfectée p21WAF1 U937 (p21-WAF1), avec l'image de la distance code du noyau.
   d) Représentation des couches successives de la taille d'un pixel, classées par leur distance par rapport à la bordure du noyau.
   e) : Reconstruction d'un volume de noyau à partir d'une image en deux dimensions. La sphère (R : rayon) a été découpée en un jeu de cylindres (rayon : r₁, hauteur : h₁, épaisseur : ΔR)
   f) : la distribution périphérique a été modélisée par distribution aléatoire dans une couronne d'épaisseur 2/10 R.
   g) : La modélisation de distribution d'un signal dans des couronnes de différentes épaisseurs selon la distance avec la bordure nucléaire.

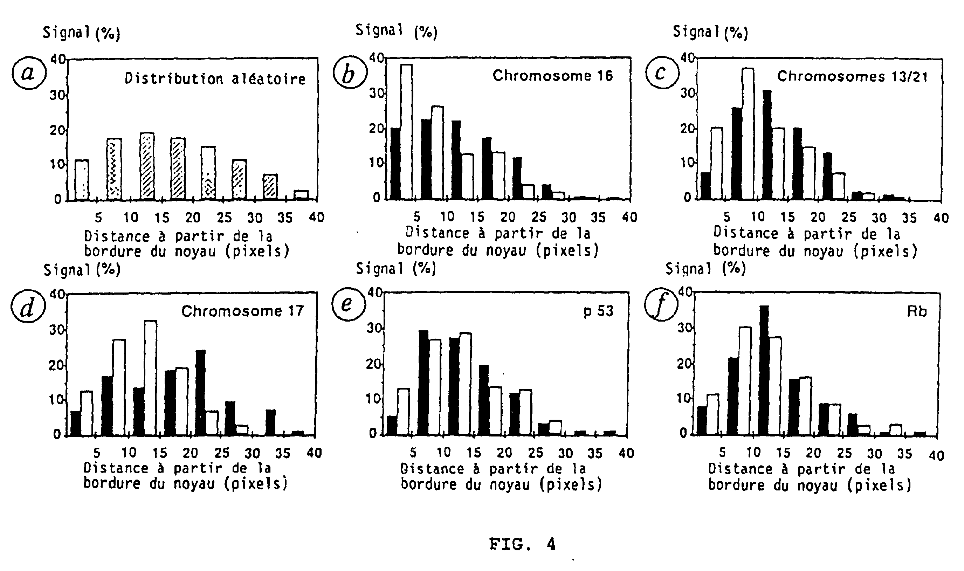

Figure 4 : Histogramme de la distribution des signaux tels que mesurés à partir de la bordure extrême du noyau.
   Comparaison entre les cellules transfectées témoin pRSV U937(■) et les cellules transfectées pRSV-p21 U937 (□).
   a : distribution de points aléatoires dans une sphère
   b-f: distribution de signaux FISH tels que mesurés à partir de la bordure du noyau pour respectivement le chromosome 16 (b), les chromosomes 13/21 (c), le chromosome 17 (d), le gène p53 (e) et le gène Rb (f).

### EXEMPLE 1

### Transfection

L'ADNc complet codant pour le gène humain p21WAF1 a été cloné dans le site EcoRI du phagemide pBK-RSV (Stratagène). Les constructions ont été transfectées en utilisant le réactant Lipofectin (Gibco-BRL). 3,5x10⁶ cellules sont incubées 8 heures avec un complexe Lipofectin (30 µg)-ADN (20 µg).

### Analyse par Northern blot

L'extraction d'ARNm et les analyses Northern blot sont effectuées en utilisant la procédure standard, la sonde p21WAF1 correspond à un ADNc de 2,1 kb Pour HUMSIAH, un fragment de 1,4 kb d'ADNc est utilisé, la sonde Rb1 est HP126 (Medgene) et la sonde Rbr-2 est un fragment PCR de 393 bp obtenu avec les amorces suivantes

5'-TGGGACAGCTACCGCAGCATGAGCGA-3'
et 5'-CACAGTACAGGGCTGTCGCCGCTGTT-3'.

### Analyse de la tumorigénicité

L'injection des souris scid/scid a été réalisée comme décrit précédemment (Telerman et al., 1993). 10⁷ cellules sont injectées par site. Les animaux sont suivis pendant trois mois. Le test de Mann-Whitney a été utilisé pour l'analyse statistique.

### Exposition du génome à la DNase I.

Le test d'exposition du génome à la DNase I a été modifié par rapport à celui décrit par Puck (Puck et al, 1991). Les cellules sont fixées dans 4 % de paraformaldéhyde/PBS déshydraté (PBS : tampon phosphate salin) et stocké à - 80°C. La nick-translaiton in situ (NT) a été réalisée en utilisant 30 U de DNase I (Boehringer Mannheim, Allemagne) dans un tampon NT (50 mM Tris HCl pH 8, 5 mM de MgCl₂. 10 mM du β-mercaptoéthanol, 50 ng/ml BSA (albumine sérique bovine)) à température ambiante et 40 U d'ADN polymérase I (Boehringer Mannheim, Allemagne), 100 mM dNTP digoxigénine mix marquée (Boehringer Mannheim, Allemagne). L'ADN in situ réparé a été révélé en utilisant des anticorps anti digoxigénine conjugués à la péroxidase (Boehringer Mannheim, Allemagne) et la tyramide-TRITC (Du Pont NEN, Etats-Unis) comme substrat. Les coupes ont été marquées avec la chromomycine A3 (Sigma, Etats-Unis). L'analyse a été réalisée par microscopie laser à balayage cofocal (MRC 600 Bio-Rad, Grande-Bretagne) en mode fluorescence.

### Hybridation par fluorescence in situ (FISH).

La FISH a été réalisée comme décrit précédemment par Linares-Cruz et al. (Linares-Cruz et al., 1995, Histochemical journal, 27, 15-23). Les sondes suivantes ont été utilisées (ONCOR, Etats-Unis) : une sonde centromérique alphoïde correspondant au chromosome 16 (D16Z2), une sonde centromérique alphoïde correspondant au chromosomique 17 (D17Z1), et des sondes centromériques alphoides correspondant aux chromosomes 13/21 (D13Z1/D21Z1), aux gènes p53 et Rb. L'analyse a été réalisée par microscopie à épifluorescence (avec une caméra 3CCD refroidi, équipée avec une bande passante triple, Lhesa France), en utilisant des lentilles de faible ouverture numerique (N.A.) (40x ; 0,75 N.A.), digitalisées dans une matrice de 768 x 512 pixels Chaque pixel correspond à 55 x 55 nm de l'objet.

### L'analyse d'image.

Les mesures de distance ont été effectuées en utilisant un système d'analyse d'image SAMBA IPS (UNILOG, France) Les marqueurs ont été extraits par transformation « top hat » et les noyaux ont été fractionnés par seuil à partir de l'image par fluorescence au DAPI. Deux images binaires ont été obtenues respectivement pour les marqueurs et pour les noyaux. L'érosion successive a été utilisée pour mesurer la distance entre les marqueurs et la bordure du noyau. Les positions des marqueurs ont été déterminées par l'intersection de leur image avec la distance. Cette méthode permet d'analyser la totalité des noyaux indépendamment de leur forme. Pour compenser les différences de taille nucléaire, les distances ont été systématiquement normalisées en divisant chaque valeur de distance par le rayon du noyau correspondant.

### Modèle de distribution des distances.

Les courbes modèles utilisées pour la comparaison des distances obtenues expérimentalement ont été réalisées par simulation stéréologique. La représentation en trois dimensions des noyaux est assimilée à un modèle sphérique formé d'un jeu de cylindre. Le volume d'un cylindre élémentaire (vᵢ) est corrélé à la probabilité (Pᵢ) de présence des marqueurs par : Pᵢ = vᵢ / Σ vᵢ. Si R est le rayon du noyau, rᵢ le rayon du cylindre, Δ R l'épaisseur du cylindre et hᵢ sa hauteur vᵢ = Π Δ R (2 rᵢ - Δ R) hᵢ.

Cette équation permet de tracer la distribution aléatoire dans une sphère et la distribution périphérique dans les couronnes de différentes épaisseur. Avec ce modèle la fonction obtenue est indépendante de la forme elliptique du noyau. Les spots FISH ajustés à un réglage de focal (plan équatorial) sont analysés. Plus de 100 noyaux ont été évalués par cas.

### Analyse statistique.

Le rayon du noyau est divisé en huit classes. On a répertorié en une de ces huit classes chaque distance mesurée entre la bordure nucléaire et les signaux FISH. Le test du X² a été utilisé pour confirmer deux hypothèses. Premièrement, que les distances mesurées dans les cellules p21WAF1 U937 sont distribuées de manière similaire que celles mesurées dans les cellules U937 transfectées avec seulement le vecteur. Deuxièmement, que les distances mesurées dans les deux lignées cellulaires sont distribuées de manière similaire comme des points aléatoires dans une sphère. P < 0,001 est considéré comme indiquant une différence significative.

### Réexpression de p21 WAF1 dans un système modèle

Les cellules transformées sont les cellules d'une lignée cellulaire tumorale U937, lignée tumorigène à la fois *in vitro* et *in vivo,* qui n'exprime pratiquement pas p21WAF1, aussi bien au niveau de l'ARNm qu'au niveau protéique (Telerman et al., 1993, et Nemani et al., 1996).

On a observé que les cellules filles US3-US4 dérivées de la lignée cellulaire tumorale U937, sélectionnées au moyen du parvovirus H-1, présentent une suppression du phénotype tumoral. Il a été montré, par exemple, que les cellules U937 forment des tumeurs au niveau de 80% des sites d'injection dans les souris scid/scid alors que les cellules US3 ne forment aucune tumeur et que les cellules US4 forment une tuneur pour 20 sites d'injection. Ces cellules US3 et US4 expriment, par contre, de façon constitutive des niveaux très élevés de p21WAF1 indépendamment de la voie p53.

C'est pourquoi, ces observations ont conduits à étudier l'effet de p21WAF1 sur l'expression de gène spécifique et la supression de tumeur de cellules U937 transfectées de façon stable par p21WAF1.
Induction par p21WAF1 de l'expression de gène spécifique et supression de la tumorigénicité

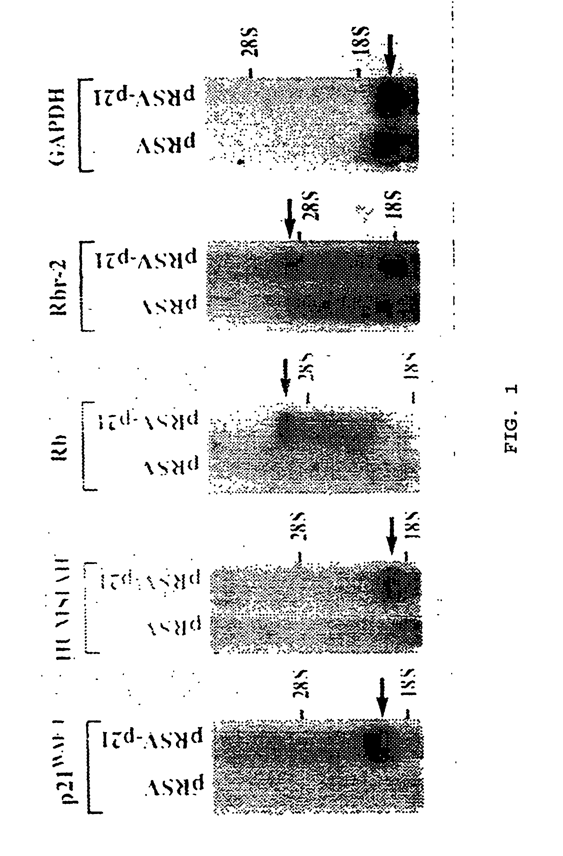

Après transfection des cellules U937 avec les vecteurs précédents, on constate après analyse par Northern blot (cf. figure1) que les cellules U937 transfectées par p21WAF1 (pRSV-p21) présentent un taux très élevé d'ARNm correspondant à l'expression du gène p21WAF1 de l'homologue humain du gène de la Drosophile "seven in absentia" (HUMSIAH) Ce gène (dénommé encore TSAP 3, tumeur supresseur activé voie 3) est activé par la protéine p53 sauvage à un stade très précoce de l'apoptose et de la suppression tumorale (Nemani et al., 1996, et Amson et al., 1996). En outre, le gène de susceptibilité au rétinoblastome Rb (Weinberg et al., 1992) et le gène codant pour la p130 en liaison avec Rb (Rbr-2)( Hannon et al., 1993, Genes and Development, 7, 2378-2391) sont également très fortement activés dans les transfectants p21 des cellules U937 (cf figure 1). L'expression du gène p53 au niveau ARNm et protéique reste non détectable à la fois dans le témoin et dans les cellules transfectées (résultats non montrés). L'expression du gène Rb établit ses propriétés suppresseurs de tumeurs, de même que pour le gène Rbr-2 pour ses implications dans le cycle cellulaire et la localisation chromosomique de HUMSIAH. Cependant, l'analyse du cycle cellulaire par FACS ( analyse par cytométrie de flux) dans trois expériences indépendantes ( en utulisant le iodure de popidium), n'a pas révéléé de différence significative entre les cellules U937 transfectées témoin et les cellules U937 p21-WAF1 (exemple de résultats obtenus : i) cellules U937 transfectées témoin: en G₁ 65%/S29%/G₂ 6% ; ii) cellules U937 p21-WAF1 : G₁ 64%/S30%/G₂ 6%).

Le temps de doublement est aussi similaire avec les deux lignées, respectivement de 33 heures pour les cellules U937 transfectées témoin et de 36 heures pour les cellules U937 p21-WAF1

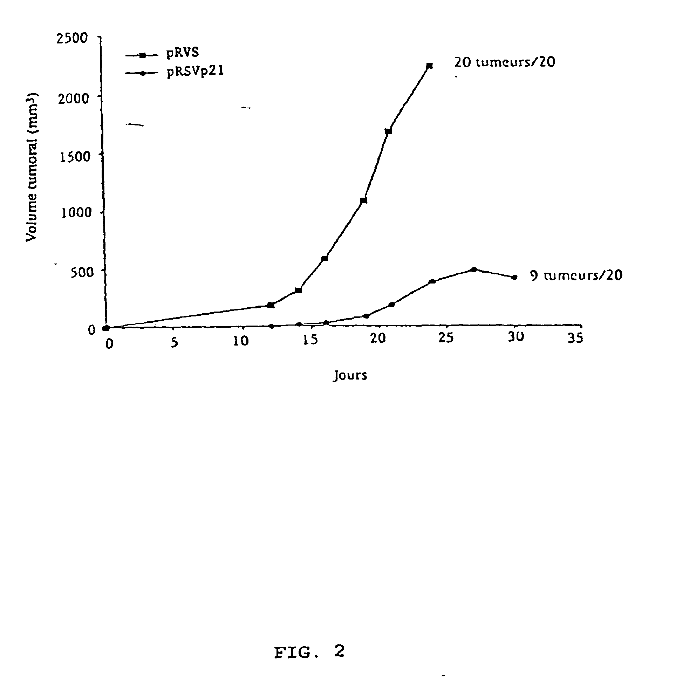

L'injection dans des souris Scid/Scid est effectuée comme cela a été décrit précédemment La comparaison est effectuée avec des cellules transfectées par le vecteur seul (pRSV). La GAPDH est utilisée comme contrôle. Après transfection des cellules U937 avec les vecteurs précédents, on constate que les cellules U937 transfectées de façon stable par p21WAF1 ( pRSV-p21) montrent une disparition significative du phénotype malin (cf. figure 2). Après 24 jours, les souris scid/scid injectées avec des cellules U937 contenant le vecteur seul présentent des tumeurs au niveau de tous les sites et d'un volume tel qu'elles ont du être sacrifiées. Au contraire, seules 9 sites sur 20 sites d'injection de cellules p21WAF1 U937 transfectées ont formés des tumeurs apparamment retardées et d'un volume beaucoup plus petit. Aucune autre tumeur n'est apparue après 6 semmaines supplémentaires d'observatuion Ainsi, l'induction spécifique de l'expression du gène p21-WAF1, sans variation importante de cycle cellulaire, est en partie responsable de l'activation du processus moléculaire de la supression de tumeur

### EXEMPLE 2

### Méthode à la DNase I - Influence de p21WAF1 sur la réorganisation du noyau

Cet exemple a pour objet d'étudier le rôle de p21WAF1 dans une possible réorganisation du noyau.

Le procédé utilisé pour cette mise en évidence du fait que la position des chromosomes dans le noyau pourrait avoir une influence sur le phénotype cellulaire, est le procédé dit à la DNase I qui permet de visualiser l'ADN exposé, c'est-à-dire des portions de chromosome qui sont plus sensibles à la DNase I dans certaines conditions, ce qui permet de les marquer.

Pour ce faire, on a repris la méthode à la DNase I telle que décrite dans le brevet US 5 264 343 mais de façon à pouvoir l'appliquer à des tissus.

Les tissus sont fixés dans un mélange à 4% de paraformaldéhyde dans PBS pendant 10 minutes, déshydratées et stockées à -80°C.

Le marquage par "nick-translation" est effectué en utilisant un tampon de "nick-translation" (NTB) et une solution 10x (100 ml).

NTB comprend 50 mM Tris HCL pH 8,5 mMgCl₂, 10 mM β-mercapto-éthanol et 50 ng/ml de BSA. La solution 10x (100 ml) contient 50 ml de Tris HCl pH 8 (1 M), 3,3 ml de MgCl₂ (1,5 M), 700 ml de β-mercaptoéthanol (14,2 M) et 1,66 µl de BSA (à 3%).

On traite les tissus avec 100 µl de NTB contenant 30 U de DNase-I pour chaque coupe. On incube pendant 3 minutes à la température ambiante, on bloque avec l'EDTA 20 mM, pH 8, pendant 3 minutes puis on lave 3 fois avec NTB

On utilise ensuite 100 µl de NTB avec 40 U de DNA polymérase I et 100 µM de dNTP digoxigénine (10% de la solution 10x pour chaque coupe). On incube pendant 6 minutes à la température ambiante et on bloque avec l'EDTA comme précédemment, on lave 3 fois avec NTB.

Le marquage est effectué après saturation avec 3% de BSA, révélation avec des anticorps anti-DIG marqués à la peroxydase, après lavage 3 fois 3 minutes avec PBS, on utilise comme substrat la thyramide- TRITC puis on lave 3 fois pendant 3 minutes avec PBS.

Les plaques sont ensuite contrecolorées avec la chromomycine 3-A.

L'analyse est effectuée par microscopie laser cofocale (MRC 600 Bio-Rad) en mode fluorescent (les résultats sont représentés par des photographies en couleur non incluses dans la présente description).

Les cellules cancéreuses U937 parentales ou les mêmes cellules transfectées avec le vecteur de contrôle présentent une apparence diffuse telle qu'elle est observée dans les biopsies de carcinomes mammaires Les cellules U937 transfectées avec p21WAF1 présentent un changement radical dans l'exposition du génome à la DNase I en présentant une apparence similaire à celle des cellules US3-US4 ayant une suppression du phénotype tumoral, de même qu'elles présentent une apparence correspondant à celle de cellules épithéliales observées sur des biopsies de seins normaux, c'est-à-dire un marquage selon une couronne à la périphérie du noyau.

Cette réorganisation de noyaux peut être associée avec l'activation de gènes dans la suppression de la tumorigénicité. Cette réorganisation du noyau, induite par p21-WAF1, pourrait soit être un effet direct de cette induction ou soit liée à sa fonction comme inhibiteur de l'activité cdk ( « Cyclin dependent kinase »). Le groupe des lamines nucléaires (Foisnier et al., 1993, Cell., 73, 1267-1279) est une des cibles caractérisés pour l'activité cdk mitotique. Les modifications nucléaires peuvent résulter de changement d'état de phosphorylation de ces lamines nucléaires. Ce changement important de la répartition des sites sensibles à la DNase I pourrait donc avoir comme origine le déclenchement des mouvements de chromosomes à l'intérieur du noyau induit par p21-WAF1.

### EXEMPLE 3

### Marquage in situ

On effectue une hybridation de l'ADN in situ avec une sonde centromérique 16 alphoïde, comme cela est décrit précédemment (Klinger K. et al Am. J. Hum. Genet. 51, 55-65 (1992)).

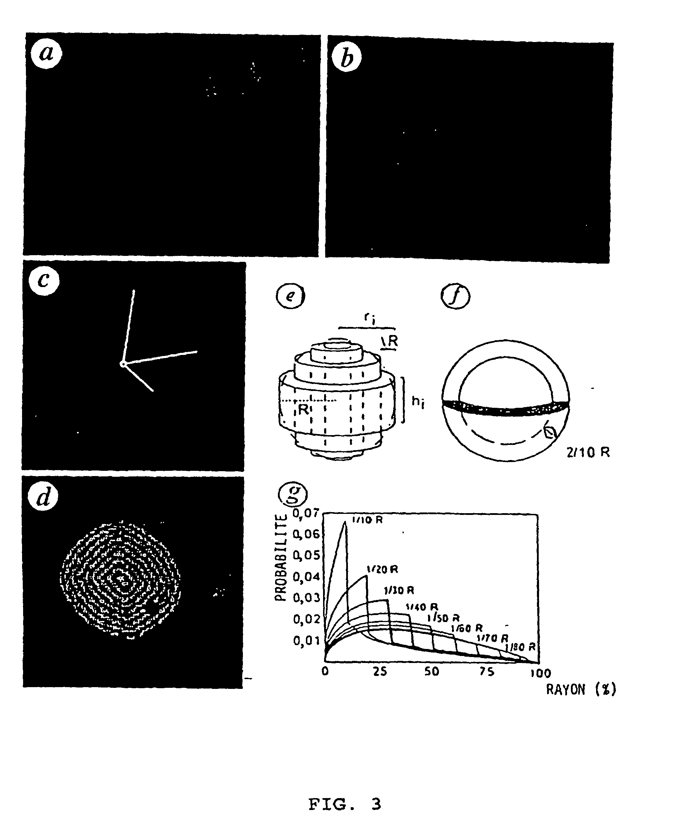

HUMSIAH et Rbr-2 étant colocalisés sur le chromosome 16q tandis que Rb est localisé sur le chromosome 13q, on utilise des sondes correspondantes. En utilisant la fluorescence par hybridation in situ (FISH), combinée avec l'optomicroscopie digitale, avec ces sondes centromériques alphoïdes correspondant au chromosome 16, on peut observer sur les photographies couleurs obtenues (en noir et blanc dans la présente description) (cf. figure 3, a)-d)) que les cellules U937 présentent un signal d'hybridation près du centre du noyau. Au contraire, les cellules U937 transfectées par p21WAF1 ou les cellules US3-US4 ayant une suppression tumorale, présentent un signal qui est détecté dans la région périphérique du noyau. Le noyau des cellules transfectées U937 témoin et les cellules transfectées p21-WAF1 sont marqués au DAPI (bleu). Le signal FISH est révélé par la tyramide-TRITC (rouge). Du point de vue physiologique, ces observations confirment l'évidence d'une organisation territoriale chromosomique à l'interphase. La conformation topologique précise du gène dans le noyau présente une répercussion fonctionnelle évidente. En particulier, cette réorganisation du noyau pourrait être responsable de l'activation des gènes impliqués dans la suppression des tumeurs, lesquels sont silencieux dans les processus tumoraux.

Pour chaque sonde utilisée, la distance à partir de la bordure du noyau a été mesurée et intégrée dans un modèle (cf. figure 3, e)-g)).

Il a été trouvé que les cellules transfectées p21-WAF1 U937 présentent une redistribution globale des signaux avec un déplacement nette vers la région périphérique du noyau (cf figure 4). La distribution de ces signaux FISH correspondant aux sondes centromériques est, de manière statistique, différente entre les cellules tumorales et celles présentant une suppression tumoral. Le test du X² est très significatif (p < 0,001). En utilisant les sondes spécifiques pour les gènes Rb and p53 nous n'observons pas de différence statistique significative dans la distribution entre les spots FISH correspondant aux cellules transfectées avec le vecteur pRSV seul et avec p21-WAF1 (en utilisant le test du X²)( cf. figure 4). D'un point de vue physiologique ces observations sont complémentaires des observations suggérant fortement l'organisation chromosomique territoriale à l'interphase (Manuelidis et al., 1990, Science, 250, 1533-1540 ; Cremer et al., 1993, Cold Spring Harbor Symposia on Quantitative Biology, LVIII, 777-792, et Puck et al., 1991)) Des domaines spécifiques de la chromatine occupent de manière distincte et non aléatoire des positions spatiales telles que décrites précédemment (Hulspas et al, 1994, Chromosoma, 103, 286-292 ; Nagele et al., 1995, Science, 270, 1831-1835). Il n'est pas certain qu'une corrélation existe entre l'expression du gène et le repositionnement du chromosome ,ceci reste à être exploré dans la mesure où certains résultats indiquent à la fois des gènes actifs et inactifs localisés préférentiellement dans les territoires périphériques des chromosomes. Notre étude montre que dans le cas d'une suppression de la tumorégénicité induite par p21-WAF1, les régions centromériques, ne contenant pas de matériel génétique codant, sont repositionnées. Ce repositionnement des régions centromériques de chromosomes analysé ci-dessus peut être à l'origine d'un mécanisme par lequel l'architecture du territoire chromosomique est modifié. De telles modifications territoriales peuvent influencer l'environnement spatial de gène spécifique et moduler leur expression. De manière évidente, la conformation topologique précise des gènes à l'intérieur du noyau devrait avoir des répercussions fonctionnelles. En effet, des travaux récents décrivant la diversité des effets de position chez la Drosophile ont clairement mis en évidence l'importance fonctionnelle de l'architecture nucléaire spécifique dans l'expression de gènes ( Csink et al., 1996, Nature, 381, 529-531 ; Dernburg et al., 1996, 85, 745-749)

En conclusion en utilisant à la fois les systèmes modèles d'analyse de la suppression de la tumorigénicité et la transfection stable, les résultats présentés ci-dessous montrent que p21-WAF1 induit le repositionnement du matériel génétique dans le noyau, associé à des fonctions potentiellement importantes pour atteindre une suppression tumorale. Ces résultats montrent le rôle central joué par p21WAF1 dans l'organisation du noyau, dans le positionnement des chromosomes et dans l'activation du processus de suppression de la tumorigénicité.

## Revendications

1. Procédé destiné à mettre en évidence l'état d'une cellule maligne, **caractérisé en ce qu'**il comprend les étapes suivantes :
a) évaluer la position d'un ou plusieurs gènes dans le noyau en évaluant la position des centromères de chromosomes qui les portent par hybridation *in situ* par fluorescence en utilisant des sondes centromériques ;
b) évaluer l'écart de cette position par rapport à une cellule normale comme index de sa malignité,
dans laquelle une localisation périphérique des chromosomes portant ces sondes centromériques correspond à la normalité.

2. Procédé selon la revendication 1, **caractérisé en ce que** le chromosome est le chromosome 13q et/ou 16q.

3. Procédé selon l'une des revendications 1 et 2, **caractérisé en ce que** l'on met en évidence l'état d'une cellule maligne, puis on traite ladite cellule avec un produit anticancéreux ou apparenté et on évalue l'activité desdits composés en mesurant la variation de l'index de malignité.

## Claims

1. Method intended for determining the state of a malignant cell, **characterized in that** it comprises the following steps:
a) evaluating the position of one or more genes in the nucleus by evaluating the position of the centromeres of chromosomes carrying them by fluorescence in situ hybridization using centromeric probes;
b) evaluating the deviation from this position compared with a normal cell as index of its malignancy, in which a peripheral location of the chromosomes carrying these centromeric probes corresponds to the normal.

2. Method according to Claim 1, **characterized in that** the chromosome is chromosome 13q and/or 16q.

3. Method according to either of Claims 1 and 2, **characterized in that** the state of a malignent cell is determined and then said cell is treated with an anticancer product or a related product and the activity of said compounds is evaluated by measuring the variation in the malignancy index.

## Patentansprüche

1. Verfahren, welches dazu bestimmt ist, den Status einer malignen Zelle nachzuweisen, **dadurch gekennzeichnet, dass** es die folgenden Schritte umfasst:
a) die Position von einem oder mehreren Genen in dem Kern auszuwerten, indem die Position der Centromeren von Chromosomen, die diese tragen, durch Fluoreszenz-in situ-Hybridisierung unter Verwendung von Centromersonden ausgewertet wird;
b) die Abweichung von dieser Position bezogen auf eine normale Zelle als Index für deren Malignität auszuwerten,
bei welcher eine periphere Lokalisierung der Chromosomen, die diese Centromersonden tragen, der Normalität entspricht.

2. Verfahren nach Anspruch 1, **dadurch gekennzeichnet, dass** das Chromosom das Chromosom 13q und/oder 16q ist.

3. Verfahren nach einem der Ansprüche 1 und 2, **dadurch gekennzeichnet, dass** man den Status einer malignen Zelle nachweist, man die Zelle dann mit einem Antikrebs-Erzeugnis oder verwandten Erzeugnis behandelt und man die Aktivität der Verbindungen auswertet, indem die Variation des Malignitätsindexes gemessen wird.
